# EUROPEAN PATENT SPECIFICATION

(11) **EP 0 341 039 B1**
(45) Date of publication and mention of the grant of the patent: **10.03.1993**
(21) Application number: 89304440.4
(22) Date of filing: 03.05.1989
(51) Int. Cl.: A61M 31/00

(54) **Transendoscopic implant capsule**
Transendoskopische Implantatkapsel
Capsule implantable transendoscopique

(30) Priority: 04.05.1988 US 190108
(43) Date of publication of application: 08.11.1989
(73) Proprietor: TRIANGLE RESEARCH AND DEVELOPMENT CORPORATION, Raleigh North Carolina (US); THE JOHNS HOPKINS UNIVERSITY, Baltimore, MD 21218 (US)
(72) Inventor: Colvin, David P., Apex North Carolina 27502 (US); Marsh, Bernard R., Upperco Maryland 21155 (US)
(74) Representative: Lees, Clifford

(56) References cited:
- EP-A- 0 174 865
- FR-A- 2 608 418
- US-A- 3 351 049
- US-A- 4 584 991

## Description

### Technical Field

The present invention relates to a device for applying a therapeutic treatment to a selected portion of the body and, more particularly, to an improved device for applying therapeutic radiation by being transported into a body orifice via a fibreoptic endoscope to a predetermined portion thereof.

### Background Art

In the United States there are presently over 100,000 deaths per year resulting from bronchial carcinoma or lung cancer. Conventional treatments for lung cancer include surgery, radiation therapy and chemotherapy. Although the surgical removal of a malignant lung tissue remains as the effective method of choice of physicians, it may only be accomplished in a few early cases before intrathoracic or distant spread of the tumor has taken place. Radiation therapy by external beam has been found to be of limited value in prolonging the life of a patient with the disease even with doses in the range of 5,000 to 6,000 rads in view of the inability to control the irradiated tumor and a high incidence of distant metastases.

Interstitial irradiation has been shown to be promising for the local treatment of certain tumors assuming that the dose rate of radiation delivered from a stereotaxically implanted source is sufficiently high that tumor cells receive a critical threshold dose during a complete cell cycle. This condition can be met by using high-activity radioisotopes which can be removed from the site after the dose is delivered thereto. If the high-activity radioisotope were to remain implanted, normal tissue surrounding the radioactive source would be exposed to potentially toxic doses of radiation. It is known to utilise removable radioactive sources encased within catheters made to precisely hold the radioactive source at the tumor target site. An improvement in this art is disclosed in U.S. Patent No. 4,584,9091 to Tokita et al.

European Patent Application No. 0,174,865 discloses a capsule for insertion into the rumen of an animal. The capsule is adapted to be retained in position within the animal by means of pivotally attached wings. This device however is not suitable for use with radiation therapy, as there are no means for easily removing the capsule once in position, leading to an over exposure to radiation.

In view of shortcomings presented by the present state of the radiation therapy art, applicant has developed an inventive implant capsule for radioisotopes which is a adapted to be remotely implanted and retrieved by means of a catheter inserted through the collateral lumen of a fibreoptic bronchoscope. The capsule possess resilient engaging means to enable the implant device to engage the bronchial walls when released at a predetermined location for treatment of a bronchial malignancy. The implant capsule has application to other organs and other types of endoscopes and may also be used for endoscopic drug delivery by placing a chemotherapeutic agent therein for localised or topical chemotherapy treatment.

### Disclosure of the Invention

In accordance with the present invention, the applicant provides a device for applying a therapeutic treatment to a selected portion of a human or animal body for a predetermined period of time comprising :
a body member defining at least one elongate therapeutic treatment material receiving chamber;
at least one resilient arm member co-operatively associated with said body member and extending radially outwardly therefrom, the or each arm member being adapted to be compressed radially inwardly during transportation of the device to the portion of the body requiring therapeutic treatment and then released so as to allow the arm member to return to its outwardly extending position in order to engage with the body portion so as to be located for applying the therapeutic treatment characterised by the or each resilient arm member extending outwardly from a central stem, the central stem being secured to said body member substantially concentrically therewith and extending substantially parallel to the treatment receiving chambers.

Accordingly, it is an object of the device of the invention to provide an improved implant capsule of the type adapted to contain a radioactive therapeutic treatment for point-source localised radiotherapy.

A further object of the present invention is to provide an implant capsule for application of radiotherapy or chemotherapy treatment which is adapted to be remotely implanted and retrieved with a fibreoptic bronchoscope.

A still further object of the present invention is to provide an implant capsule adapted for containing a selected number of radioisotope seeds for radiotherapy which can be remotely implanted and retrieved with a fibreoptic bronchoscope and which is further adapted to engaged the body portion with resilient arms when implanted therein.

### Brief Description of the Drawings

Some of the objects of the invention having been stated, other objects will become evident as the description proceeds, when taken in connection with the accompanying drawings, in which :-
Figure 1 is an exploded perspective view of a preferred embodiment of the implant capsule of the present invention;
Figure 1A is a view of the body member of the present invention taken along line 1A - 1A of Figure 1;
Figure 2 is a side elevation view of the implant capsule shown in Figure 1 with the resilient arm members fully extended;
Figure 3 is a side elevation of the implant capsule of Figure 1 with the resilient arm members fully collapsed within the body member thereof;
Figure 4 is a perspective view of a second embodiment of the implant capsule of the present invention comprising a shortened body member;
Figure 5 is a perspective view of a third embodiment of the implant capsule of the present invention comprising a shortened body member and a flexible central stem from which the arm members extend; and
Figure 6 is a perspective view of another embodiment of the implant capsule of the present invention wherein the body member comprises a singular flexible elongate chamber.

### Best Mode for Carrying Out the Invention

Referring now generally to the drawings, several different embodiments of an implant capsule of the present invention are depicted which comprises a body member defining at least one elongate therapeutic treatment material receiving chamber therein to hold radioisotope seeds or a chemotherapeutic agent and resilient arm members to restrain movement of the capsule when properly position within the body. The implant capsule's resilient arm members are designed to have maximum stored spring energy per unit length in order to minimise the required size of the capsule when retracted and released using an external catheter and forceps which can be introduced through a fibreoptic bronchoscope. A standard biopsy-type forcep modified to grasp the spherical tip of the stem of the resilient arm members may be utilised within a sheath to retract the resilient arm members by sliding the sheath thereover and later to extend them by removing the sheath. Although the use of a catheter or sheath with a slidably movable biopsy forcep therein is presently contemplated as the preferred type of instrument to manipulate the implant capsule of the present invention, other manipulation means are clearly possible.

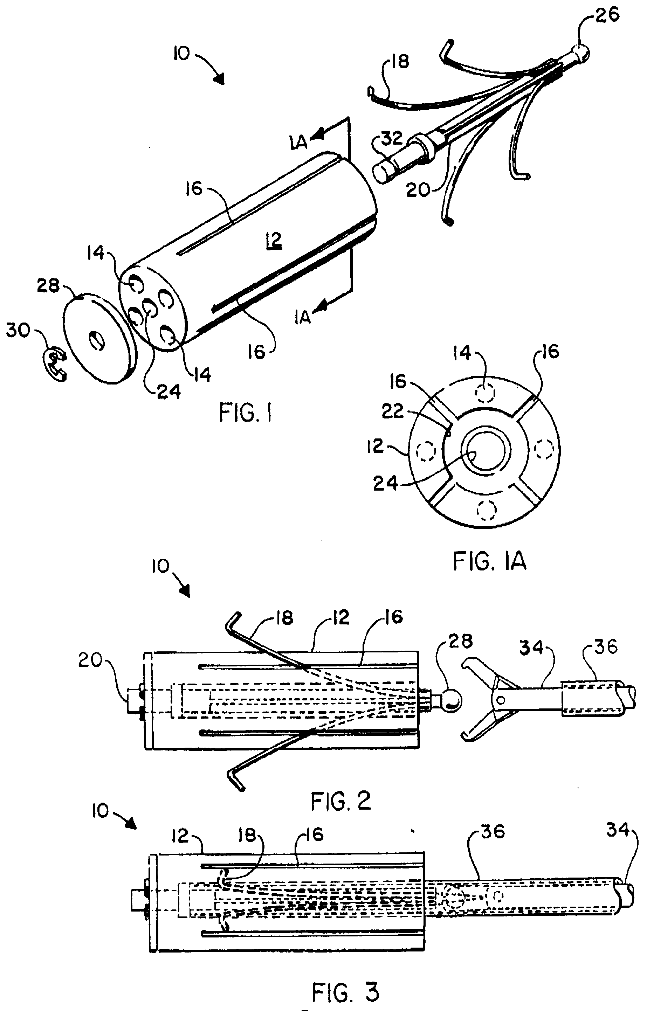

Referring now specifically to Figures 1 to 3, there is illustrated a preferred embodiment, generally designated 10, of the implant capsule of the invention for applying therapeutic radiation to a selected portion of the body. Implant capsule 10 consists of body member 12 which defines a plurality of chambers 14 therein adapted to receive a therapeutic treatment material such as radioisotope seeds and/or chemotherapeutic agents. The use of both a radioactive isotope and a chemotherapeutic material is known to have synergistic effect in the treatment of cancer, and the use of both in capsule 10 may be desirable. Body member 12 also defines a plurality of radially extending slots 16, as best seen in Figures 1 and 1A. Resilient arm members 18 are secured to central stem 20 and adapted to be slidable received by the bore 22 (see Figure 1A) and slots 16 within body member 12. The face of body member 12, as best illustrated in Figure 1A, illustrates that bore 22 extends along the length of body member 12 and terminates with a relatively small diameter aperture 24 at the remote end thereof. Central stem 20 is received within body member 12 so that the end portion thereof (opposite sphere 26) extends through aperture 24 and cap 28 so that E-clip 30 may be secured to groove 32 of the stem in order to assure a snug fit of body member 12, cap 28 and central stem 20 after a treatment material is placed within chamber 14. Although a matter of design choice, chambers 14 will accommodate up to 28 radioactive isotope units and/or chemotherapeutic agent boluses in the preferred embodiment of the invention illustrated in Figures 1 to 3. Arm members 18 are most suitably fabricated from a special alloy wire (such as MP 35N manufactured by Maryland Spaciality Wire Company) having a tensile strength of at least 2,068 MNm⁻² (300,00 psi) and which is compatible with the body tissues and fluids. Central stem 20 can be stainless steel or other compatible metal and arm members 18 are attached thereto by a suitable method of fixation such as electric fusion welding. Body member 12 and cap 28 are fabricated from a biocompatible plastic material.

As best seen in Figures 2 and 3, in clinical usage a grasping forcep 34 within catheter 36 would be inserted through the collateral lumen at the proximal end of a bronchoscope (not shown) and passed therethrough to engage sphere 26 of implant capsule 10 at the remote or distal end of the bronchoscope. Flexible sheath or catheter 36 is then extended (see Figure 3) into bore 22 of body member 12 so as to extend over and collapse resilient arm members 18 into slots 16. Implant capsule 10 and catheter 36 positioned in the lumen of the fibreoptic bronchoscope are then inserted into a selected area of the patient's lung. Catheter 36 is retracted so as to permit resilient arm members 18 to extend outwardly through slots 16 and the barbs at the ends thereof to secure the capsule to the inner bronchus wall. With this retrograde-load technique, capsules larger than the accessory lumen can be implanted and retrieved. Once the capsule has been secured, the bronchoscope and associated forcep 34 and catheter 36 are temporarily withdrawn. After a predetermined prescribed treatment time, the bronchoscope is reinserted into the bronchus and the process repeated in order to retract arm members 18 and withdrawn implant capsule 10 from its location in a patient's lung.

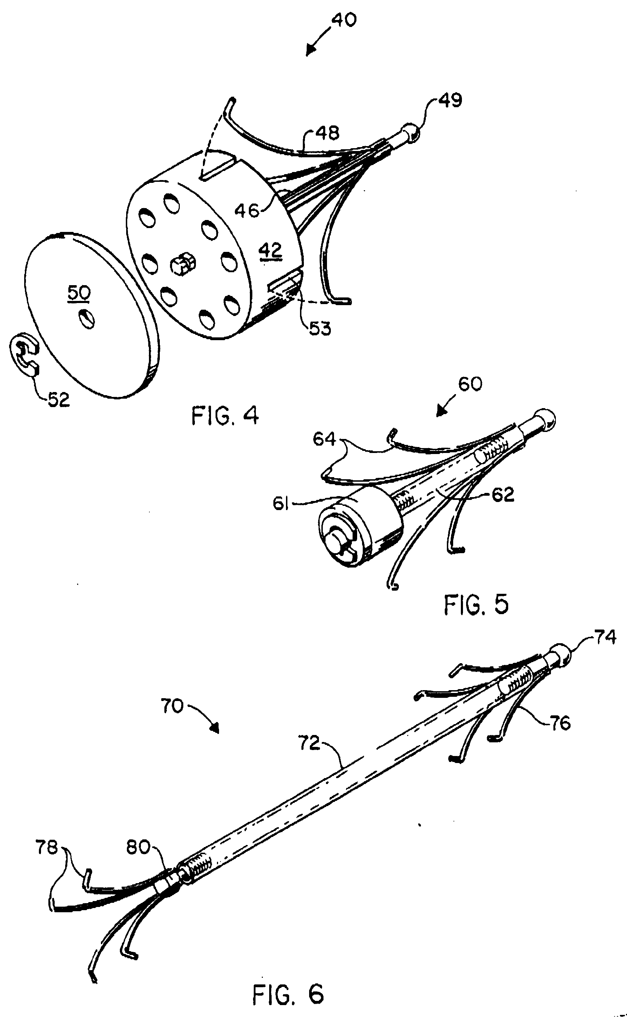

A second embodiment of the present invention is illustrated in Figure 4 and generally designated 40. Implant capsule 40 comprises body member 42 defining treatment receiving chambers 44 therein, central stem 46 with resilient arm members 48 and sphere 49, cap 50 and E-clip 52. Arm members 48 are received by slots 53 when collapsed by catheter 36 (see Figure 2) into body member 42 for insertion or removal from the lung. This embodiment of the invention is substantially similar to that shown in Figures 1 to 3 except body member 42 only extends for a portion of the length of the stem 46 and the chambers therein are of a shorter length than chambers 14 of implant capsule 10. Implant capsule 40 is therefore capable of holding fewer isotope seeds, most suitable about 8, and less of other chemotherapeutic agents than is the capsule described hereinbefore.

A third embodiment of the present invention is depicted in Figure 5 and generally designated 60. Implant capsule 60 is similar to implant capsule 40 but additionally provides a flexible central stem 62 to which resilient arm members 64 are attached. Flexible central stem 62 allows for greater ease of manipulation or flexing of implant capsule 60 as it is moved within the lumen of a bronchoscope and can also be used to contain a linear distribution of radioisotope seeds. The body or pod member 61 is smaller and presently contemplated to contain only about 4 radioisotope seeds within the chambers defined therein (not shown).

Figure 6 depicts still another embodiment of the implant capsule of the present invention, generally designated 70. Implant capsule 70 is designed to overcome certain limitations of the implant capsules illustrated in Figures 1 to 5. The relatively more rigid implant capsules described above are particularly suitable for point-source radiation to certain locations within a lung but do not lend themselves as well as implant capsule 70 to placement in an area to be irradiated which extends linearly or curvilinearly along a bronchus or around a corner thereof. For these particular types of applications, implant capsule 70 is particularly appropriate since it comprises a linear flexible body member 72, most suitably constructed of a flexible plastic, which defines a singular chamber therein for receiving a linear array of radioisotope seeds. A sphere 74 with radially extending arms 76 secured thereto (to facilitate grasping of the sphere by forceps 34) is attached to one end of linear body member 72, and arm members 78 secured to stem 80 are attached at the other end thereof to engage the bronchus wall. It should be observed that the hooks of arm members 76 extend inwardly in order to allow catheter 36 to extend thereover and slidably receive implant capsule 70 therein for placement or removal from a selected location in a lung. It should also be noted that small diameter of implant capsule 70 can permit insertion directly through a bronchoscope without the requirement of retrograde loading as for capsules described hereinbefore.

Although the implant capsules of the present invention have been discussed primarily in terms of a containment vessel for radioisotope seeds, chemotherapeutic agents, as noted hereinbefore, are also contemplated to be placed therein for localised or topical chemotherapy treatment. Though not illustrated in the drawings, the chemotherapeutic agent could be placed within an implant capsule of the present invention and released through a contact wick system, semi-permeable membrane or the like into adjacent tissue.

It will be understood that various details of the invention may be changed without departing from the scope of the invention. Furthermore, the foregoing description is for the purpose of illustration only, and not for the purpose of limitation - the invention being defined by the Claims.

## Claims

1. A device for applying a therapeutic treatment to a selected portion of a human or animal body for a predetermined period of time comprising :
a body member (12) (42) (72) defining at least one elongate therapeutic treatment material receiving chamber (14):
at least one resilient arm member (18) (48) (76) cooperatively associated with said body member and extending radially outwardly therefrom, the or each arm member being adapted to be compressed radially inwardly during transportation of the device to the portion of the body requiring therapeutic treatment and then released so as to allow the arm member to return to its outwardly extending position in order to engage with the body portion so as to be located for applying the therapeutic treatment, characterised by the, or each, resilient arm members (18) (48) (76) extending outwardly from a central stem (20) the central stem being secured to said body member substantially concentrically therewith and extending substantially parallel to the treatment receiving chamber.

2. A device according to Claim 1, wherein the body member defines a plurality of substantially parallel treatment receiving chambers (14).

3. A device according to Claim 1 or 2, wherein each of the arm members (18) (48) (76) comprises an end portion turned back on the arm to define a barb.

4. A device according to any one of Claims 1 to 3 wherein the body member is coextensive with one end of the central stem.

5. A device according to any one of Claims 1 to 4 wherein the body member is coextensive with substantially the entire length of the central stem, and the body member defines a plurality of slots (16) (53) therein each adapted to receive a corresponding resilient arm member when said resilient arm member is compressed.

6. A device according to any one of Claims 1 to 5, wherein the body member is flexible and defines a single elongated therapeutic treatment receiving chamber.

7. A device according to Claim 6, wherein there is a plurality of resilient arm members extending from a central stem which is secured to one end of the flexible body member.

8. A device according to any one of Claims 1 to 7, wherein the device houses one or more radioisotope seeds within the or each therapeutic treatment receiving chamber.

9. A device according to any one of Claims 1 to 7, wherein the device houses a chemotherapeutic agent within the or each therapeutic treatment receiving chamber.

10. A device according to Claim 9 including means for applying the chemotherapeutic agent from the or each chamber to a selected body portion.

11. A device according to Claim 9, wherein each device houses a chemotherapeutic agent and a radioactive isotope within the or each therapeutic treatment receiving chamber.

## Patentansprüche

1. Vorrichtung zum Anwenden einer therapeutischen Behandlung auf einen ausgewählten Bereich eines menschlichen oder tierischen Körpers während einer vorgegebenen Zeitspanne, umfassend:
ein Gehäuse (12, 42, 72), das wenigstens eine längliche Kammer (14) zur Aufnahme therapeutischen Behandlungsmaterials bildet;
wenigstens einen elastischen Hebelarm (18, 48, 76), der mit dem Gehäuse in Wirkverbindung steht und sich von diesem aus radial nach außen erstreckt, und der derart gestaltet ist, daß er während des Transportes der Vorrichtung zu dem Bereich des Körpers, welcher therapeutische Behandlung erfordert, radial nach innen gedrückt wird, und sodann freigegeben wird, um ihm zu erlauben, in seine sich nach außen erstreckende Position zurückzubewegen, um den Körperbereich zu erfassen, um zwecks Anwendens der therapeutischen Behandlung lokalisiert zu werden,
dadurch gekennzeichnet, daß der oder jeder elastische Hebelarm (18, 48, 76) sich von einem zentralen Schaft (20) nach außen erstreckt, und daß der zentrale Schaft am Gehäuse im wesentlichen konzentrisch hierzu befestigt ist und sich im wesentlichen parallel zur Behandlungsaufnahmekammer erstreckt.

2. Vorrichtung nach Anspruch 1, dadurch gekennzeichnet, daß das Gehäuse eine Mehrzahl von im wesentlichen parallel angeordneten Behandlungsaufnahmekammern (14) definiert.

3. Vorrichtung nach Anspruch 1 oder 2, dadurch gekennzeichnet, daß die Hebelarme (18, 48, 76) einen Endbereich aufweisen, der am Arm zurückgekröpft ist, um einen Widerhaken zu bilden.

4. Vorrichtung nach einem der Ansprüche 1 bis 3, dadurch gekennzeichnet, daß sich das Gehäuse in gleicher Richtung wie ein Ende des zentralen Schaftes erstreckt.

5. Vorrichtung nach einem der Ansprüche 1 bis 4, dadurch gekennzeichnet, daß sich das Gehäuse im wesentlichen über die gesamte Länge des zentralen Schaftes erstreckt, und eine Mehrzahl von Schlitzen (16, 53) aufweist, deren jede dazu dient, einen entsprechenden elastischen Hebelarm dann aufzunehmen, wenn dieser zusammengedrückt wird.

6. Vorrichtung nach einem der Ansprüche 1 bis 5, dadurch gekennzeichnet, daß das Gehäuse flexibel ist und eine einzige längliche Kammer zur Aufnahme therapeutischen Behandlungsmittels bildet.

7. Vorrichtung nach Anspruch 6, dadurch gekennzeichnet, daß sich eine Mehrzahl elastischer Arme von einem zentralen Schaft aus erstreckt, der an einem Ende des flexiblen Gehäuses befestigt ist.

8. Vorrischtung nach einem der Ansprüche 1 bis 7, dadurch gekennzeichnet, daß das Gehäuse eine oder mehrere Radioisotopenquellen innerhalb der oder jeder therapeutischen Behandlungskammer aufnimmt.

9. Vorrichtung nach einem der Ansprüche 1 bis 7, dadurch gekennzeichnet, daß die Vorrichtung innerhalb der einzelnen therapeutischen Behandlungskammern eine chemotherapeutischen Wirkstoff enthält.

10. Vorrichtung nach Anspruch 9, dadurch gekennzeichnet, daß Mittel zum Aufbringen eines chemotherapeutischen Wirkstoffes aus der einzelnen Kammer auf einen ausgewählten Körperbereich vorgesehen sind.

11. Vorrichtung nach Anspruch 9, dadurch gekennzeichnet, daß jede Vorrichtung einen chemotherapeutischen Wirkstoff sowie ein radioaktives Isotop innerhalb der einzelnen therapeutischen behandlungsaufnehmenden Kammer enthält.

## Revendications

1. Dispositif pour appliquer un traitement thérapeutique à une partie sélectionnée d'un corps humain ou animal pendant une période de temps prédéterminée, comportant :
un élément formant corps (12) (42) (72) qui définit au moins une chambre allongée de réception de substance de traitement thérapeutique (14);
au moins un élément formant bras élastique (18) (48) (76) qui est associé audit élément formant corps de manière à coopérer avec lui et s'étend radialement vers l'extérieur de celui-ci, chaque élément formant bras étant adapté pour être comprimé radialement vers l'intérieur pendant un acheminement du dispositf jusqu'à la partie du corps nécessitant un traitement thérapeutique et puis relâché afin de permettre à l'élément formant bras de revenir à sa position étendue vers l'extérieur en vue de venir en contact avec la partie du corps de façon à être positionné pour appliquer le traitement thérapeutique, caractérisé en ce que le ou les éléments formant bras élastiques (18) (48) (76) s'étendent vers l'extérieur depuis une tige centrale (20), la tige centrale étant assujettie audit élément formant corps sensiblement concentriquement avec celui-ci et s'étendant sensiblement parallèlement à la chambre de réception de traitement.

2. Dispositif selon la revendication 1, dans lequel l'élément formant corps définit plusieurs chambres de réception de traitement sensiblement parallèles (14).

3. Dispositif selon la revendication 1 ou 2, dans lequel chacun des éléments formant bras (18) (48) (76) comporte une partie d'extrémité rabattue sur le bras afin de définir une barbe.

4. Dispositif selon l'une quelconque des revendications 1 à 3, dans lequel l'élément formant corps est de même étendue que l'une des extrémités de la tige centrale.

5. Dispositif selon l'une quelconque des revendications 1 à 4, dans lequel l'élément formant corps est de même étendue que sensiblement la totalité de la longueur de la tige centrale, et l'élément formant corps définit en lui plusieurs fentes (16) (53) respectivement adaptées pour recevoir un élément formant bras élastique correspondant, lorsque ledit élément formant bras élastique est comprimé.

6. Dispositif selon l'une quelconque des revendications 1 à 5, dans lequel l'élément formant corps est flexible et définit une seule chambre allongée de réception de traitement thérapeutique.

7. Dispositif selon la revendication 6, dans lequel il est prévu plusieurs éléments formant bras élastiques qui s'étendent depuis une tige centrale assujettie à l'une des extrémités de l'élément formant corps flexible.

8. Dispositif selon l'une quelconque des revendications 1 à 7, dans lequel le dispositif loge un ou plusieurs germes de radio-isotope à l'intérieur de chaque chambre de réception de traitement thérapeutique.

9. Dispositif selon l'une quelconque des revendications 1 à 7, dans lequel le dispositif loge un agent chimiothérapique à l'intérieur de chaque chambre de réception de traitement thérapeutique.

10. Dispositif selon la revendication 9, comportant des moyens pour appliquer l'agent chimiothérapique provenant de chaque chambre à une partie sélectionnée du corps.

11. Dispositif selon la revendication 9, dans lequel chaque dispositif loge un agent chimiothérapique et un isotope radioactif à l'intérieur de chaque chambre de réception de traitement thérapeutique.
